(19) Europäisches Patentamt
European Patent Office
Office européen des brevets

(11) EP 2 356 988 A1

(12) EUROPEAN PATENT APPLICATION
published in accordance with Art. 153(4) EPC

(43) Date of publication:
17.08.2011 Bulletin 2011/33

(21) Application number: 09827560.5

(22) Date of filing: 18.11.2009

(51) Int Cl.:
*A61K 31/4545* (2006.01) *A61K 31/5395* (2006.01)
*A61P 27/02* (2006.01)

(86) International application number:
PCT/JP2009/069526

(87) International publication number:
WO 2010/058779 (27.05.2010 Gazette 2010/21)

(84) Designated Contracting States:
AT BE BG CH CY CZ DE DK EE ES FI FR GB GR HR HU IE IS IT LI LT LU LV MC MK MT NL NO PL PT RO SE SI SK SM TR

(30) Priority: 18.11.2008 JP 2008294022

(71) Applicant: Santen Pharmaceutical Co., Ltd
Osaka-shi
Osaka 533-8651 (JP)

(72) Inventors:
- HIRAI, Shin-ichiro
Ikoma-shi
Nara 630-0101 (JP)
- YOSHIDA, Atsushi
Ikoma-shi
Nara 630-0101 (JP)

(74) Representative: Müller-Boré & Partner
Patentanwälte
Grafinger Straße 2
81671 München (DE)

# (54) THERAPEUTIC AGENT FOR CHORIORETINAL DEGENERATIVE DISEASES COMPRISING PYRIDINE-3-CARBALDEHYDE O-(PIPERIDIN-1-YL-PROPYL)-OXIME DERIVATIVE AS ACTIVE INGREDIENT

(57) Provided is a novel prophylactic or therapeutic agent for a chorioretinal degenerative disease. The compound of formula (1) or its salt has inhibitory effects on photoreceptor cell death or visual cell death in a mouse model of light damage. Therefore, the compound or its salt is useful as a prophylactic or therapeutic agent for a chorioretinal degenerative disease such as age-related macular degeneration, retinitis pigmentosa. In the formula(1), A is a group (p1), (p2) or (p3); $R^1$ is H, alkyl, aralkyl or OH or its ester; $R^2$ is H or alkyl; $R^3$ is halogen, H, alkyl or OH or its ester; $R^4$ is halogen, H, OH, alkoxy, amino, alkylamino or cycloalkylamino, $R^3$ and $R^4$ may be joined to each other through N to form an unsaturated [1,2,4]oxadiazine ring; $R^5$ is H, alkyl or cycloalkyl; m is 0 or 1; and n is 0 or 1.

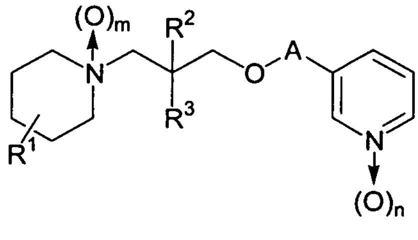

(1)

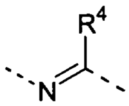

(p1)

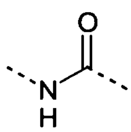

(p2)

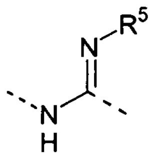

(p3)

EP 2 356 988 A1

**Description**

Technical Field

The present invention relates to a prophylactic or therapeutic agent for a chorioretinal degenerative disease containing at least one of a pyridine-3-carbaldehyde O-(piperidin-1-yl-propyl)-oxime derivative or a salt thereof as an active ingredient. The derivative has an inhibitory effect on photoreceptor cell death and/or visual cell death in a mouse model of light damage and therefore is useful as a prophylactic or therapeutic agent for a chorioretinal degenerative disease such as age-related macular degeneration or retinitis pigmentosa.

Background Art

Many chorioretinal degenerative diseases in posterior ocular tissues are intractable, and not a few chorioretinal degenerative diseases exhibit serious symptoms causing blindness. Representative examples of the chorioretinal degenerative disease include age-related macular degeneration and retinitis pigmentosa, and in particular, age-related macular degeneration is one of the leading causes of blindness in late middle to old age in developed countries such as Japan, USA, and Europe.

The age-related macular degeneration is a disease caused by age-related changes in the macular area, and at present when the population has been aging, the number of patients with age-related macular degeneration is increasing steadily. Age-related macular degeneration is broadly divided into an atrophic form (dry type) which is not accompanied by neovascularization from the choroid and an exudative form (wet type) which is accompanied by neovascularization from the choroid. For the former atrophic form, there has been no effective therapeutic method so far, and also for the latter exudative form, although laser photocoagulation, neovascular surgical removal, subfoveal translocation, or the like is performed, these treatments have limitations, and the development of an effective pharmaceutical agent has been demanded.

On the other hand, WO 00/50403 discloses N-(2-hydroxy-3-(1-piperidinyl)-propoxy)-pyridine-1-oxide-3-carboximidoyl chloride and N-((2R)-2-hydroxy-3-(1-piperidinyl)propoxy)-pyridine-1-oxide-3-carboximidoyl chloride (Arimoclomol), each of which is a compound represented by the general formula (1), and describes the use thereof as a therapeutic agent for diabetes and diabetic complication having an effect of reducing insulin resistance. WO 90/04584 discloses N-(2-hydroxy-3-(1-piperidinyl)propoxy)-pyridine-3-carboximidoyl chloride (Bimoclomol) which is a compound represented by the general formula (1), and describes the therapeutic use thereof for diabetic vascular abnormalities. WO 00/35914 discloses (-)-5-(piperidin-1-ylmethyl)-3-(pyridin-3-yl)-5,6-dihydro-2H-1,2,4-oxadiazine (Iroxanadine) which is a compound represented by the general formula (1), and describes the use thereof as a therapeutic agent for vascular diseases and diseases associated with vascular abnormalities. US Patent No. 4187220 discloses a N-(2-hydroxy-3-piperidin-1-yl-propoxy)-nicotinamidine derivative which is a compound represented by the general formula (1) shown later, and describes the therapeutic use thereof for diabetic vascular disorders. WO 2005/041965 describes the therapeutic use of N-(2-hydroxy-3-(1-piperidinyl)propoxy)-pyridine-1-oxide-3-carboximidoyl chloride and N-((2R)-2-hydroxy-3-(1-piperidinyl)propoxy)-pyridine-1-oxide-3-carboximidoyl chloride (Arimoclomol), each of which is a compound represented by the general formula (1), for neurodegenerative diseases (such as amyotrophic lateral sclerosis, Alzheimer’s disease, Parkinson’s disease, and multiple sclerosis).

However, the prophylactic or therapeutic effect of a pyridine-3-carbaldehyde O-(piperidin-1-yl-propyl)-oxime derivative represented by the general formula (1) on a chorioretinal degenerative disease such as age-related macular degeneration or retinitis pigmentosa has not been known at all.

Disclosure of the Invention

Problems that the Invention is to Solve

Accordingly, it is a very interesting object to find a new pharmaceutical use of a pyridine-3-carbaldehyde O-(piperidin-1-yl-propyl)-oxime derivative or a salt thereof.

Means for Solving the Problems

The present inventors made intensive studies to find a novel pharmaceutical use of a pyridine-3-carbaldehyde O-(piperidin-1-yl-propyl)-oxime derivative or a salt thereof. As a result, they found that the derivative or a salt thereof has an inhibitory effect on photoreceptor cell death and/or visual cell death in a mouse model of light damage and completed the invention.

That is, the invention is directed to a prophylactic or therapeutic agent for a chorioretinal degenerative disease,

particularly to a prophylactic or therapeutic agent for age-related macular degeneration, retinitis pigmentosa, or the like, containing at least one of a compound represented by the following general formula (1) or a salt thereof (hereinafter also referred to as "present compound") as an active ingredient.

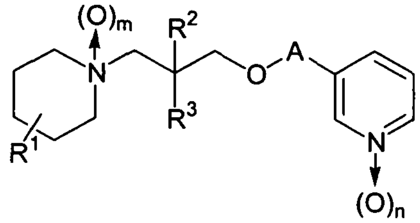

(1)

In the formula, A represents a group represented by the following general formula (p1), (p2) or (p3);

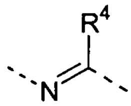

(p1)

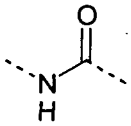

(p2)

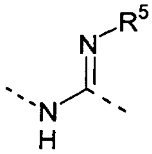

(p3)

$R^1$ represents a hydrogen atom, a lower alkyl group, an aralkyl group, or a hydroxy group or an ester thereof;
$R^2$ represents a hydrogen atom or a lower alkyl group;
$R^3$ represents a halogen atom, a hydrogen atom, a lower alkyl group, or a hydroxy group or an ester thereof;
$R^4$ represents a halogen atom, a hydrogen atom, a hydroxy group, a lower alkoxy group, an amino group, a lower alkylamino group, or a lower cycloalkylamino group; or
$R^3$ and $R^4$ may be joined to each other through a nitrogen atom to form an unsaturated [1,2,4]oxadiazine ring;
$R^5$ represents a hydrogen atom, a lower alkyl group, or a lower cycloalkyl group;
m represents 0 or 1; and
n represents 0 or 1. Hereinafter, the same shall apply.

Hereinafter, definitions of terms and phrases (atoms, groups, and the like) to be used in the specification will be described in detail.

The "halogen atom" refers to a fluorine, chlorine, bromine, or iodide atom.

The "lower alkyl group" refers to a straight-chain or branched alkyl group having 1 to 8, preferably 1 to 6, particularly preferably 1 to 4 carbon atoms. Specific examples thereof include methyl, ethyl, n-propyl, n-butyl, n-pentyl, n-hexyl, n-heptyl, n-octyl, isopropyl, isobutyl, sec-butyl, tert-butyl, and isopentyl groups.

The "lower cycloalkyl group" refers to a cycloalkyl group having 3 to 8, preferably 3 to 6 carbon atoms. Specific examples thereof include cyclopropyl, cyclobutyl, cyclopentyl, cyclohexyl, cycloheptyl, and cyclooctyl groups.

The "aryl group" refers to a residue formed by removing one hydrogen atom from monocyclic aromatic hydrocarbon, or bicyclic or tricyclic condensed polycyclic aromatic hydrocarbon having 6 to 14 carbon atoms. Specific examples thereof include phenyl, naphthyl, anthryl, and phenanthryl groups.

The "lower alkoxy group" refers to a group formed by substituting the hydrogen atom of a hydroxy group with a lower alkyl group. Specific examples thereof include methoxy, ethoxy, n-propoxy, n-butoxy, n-pentyloxy, n-hexyloxy, n-heptyloxy, n-octyloxy, isopropoxy, isobutoxy, sec-butoxy, tert-butoxy, and isopentyloxy groups.

The "aralkyl group" refers to a group formed by substituting one or a plurality of hydrogen atoms of a lower alkyl group with an aryl group which may have a substituent (here, the "substituent" means one or a plurality of halogen atoms). Specific examples thereof include benzyl, phenetyl, and naphthylmethyl groups.

The "lower alkylamino group" refers to a group formed by substituting one or two hydrogen atoms of an amino group with a lower alkyl group. Specific examples thereof include methylamino, ethylamino, propylamino, dimethylamino, diethylamino, and ethyl(methyl)amino groups.

The "lower cycloalkylamino group" refers to a group formed by substituting one or two hydrogen atoms of an amino group with a lower cycloalkyl group. Specific examples thereof include cyclopropylamino, cyclobutylamino, cyclopentylamino, cyclohexylamino, cycloheptylamino, cyclooctylamino, dicyclopropylamino, and cyclopropyl(methyl)amino groups.

The "ester of a hydroxy group" means a group represented by the following general formula (2).

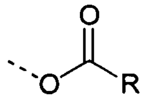 (2)

In the formula, R represents an alkyl group having 1 to 15 carbon atoms, an aryl group which may have a substituent (here, the "substituent" means one or a plurality of groups or atoms selected from a halogen atom, a lower alkoxy group, and a trifluoromethyl group), a thienyl group, a furyl group, or a pyridyl group.

Specific examples of the ester of a hydroxy group include methylcarbonyloxy, heptadecanylcarbonyloxy, 2-chlorophenylcarbonyloxy, 4-methoxyphenylcarbonyloxy, 3-trifluoromethylcarbonyloxy, thienylcarbonyloxy, furylcarbonyloxy, and pyridylcarbonyloxy groups.

The "unsaturated [1,2,4]oxadiazine" refers to a group represented by the following formula (3), (4), (5), (6), (7), or (8).

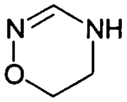 (3) 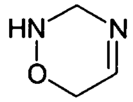 (4) 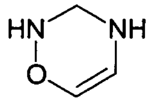 (5)

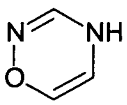 (6) 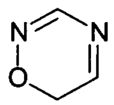 (7) 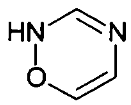 (8)

In the case where there are geometric isomers and/or optical isomers in the present compound, such isomers are also included in the scope of the present compound.

In the case where there is proton tautomerism in the present compound, the tautomers thereof (a keto form and an enol form) are also included in the scope of the present compound.

For example, in the general formula (1), when $R^4$ is a hydroxy group, it is assumed that there are proton tautomers as shown below, and these tautomers are also included in the scope of the present compound.

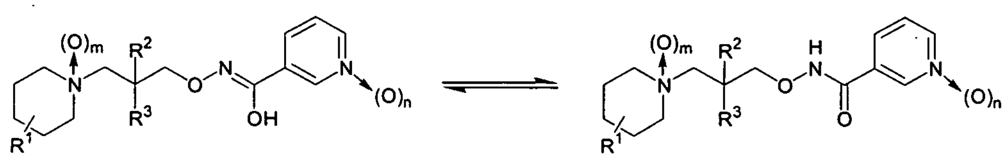

Further, when $R^4$ is an amino group, a lower alkylamino group, or a lower cycloalkylamino group, and an amino moiety thereof has one or more hydrogen atoms, it is assumed that there are tautomers as shown below, and these tautomers are also included in the scope of the present compound.

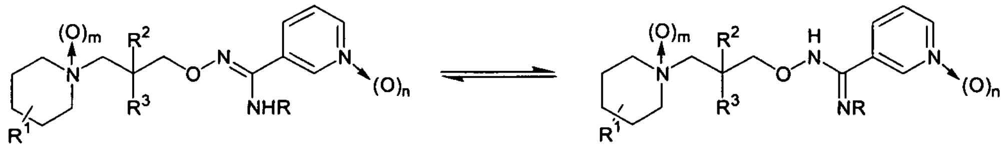

The expression "N→O" in the general formula (1) of the present compound can also be expressed as "$N^+$-$O^-$".

In the case where there are hydrates and/or solvates in the present compound, such hydrates and/or solvates are also included in the scope of the present compound.

In the case where there are crystalline polymorphisms and crystalline polymorphism groups (crystalline polymorphism systems) in the present compound, such crystalline polymorphisms and crystalline polymorphism groups (crystalline polymorphism systems) thereof are also included in the scope of the present compound. Here, the crystalline polymorphism groups (crystalline polymorphism systems) mean individual crystal forms in respective stages when the crystal forms are changed variously by conditions for the production, crystallization, storage, or the like of the crystals thereof and states thereof (the states also include a formulated state) and all the processes thereof.

(a) Examples of the present compound include compounds and salts thereof in which the respective groups are groups described below in the compounds represented by the following general formula (1) and salts thereof:

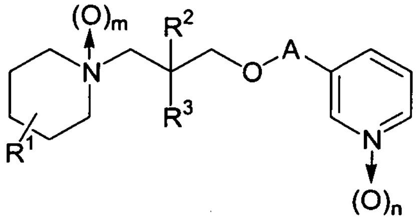
(1)

(a1) A represents a group represented by the following general formula (p1), (p2) or (p3); and/or

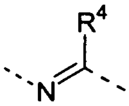
(p1)
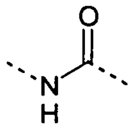
(p2)
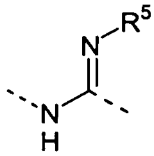
(p3)

(a2) $R^1$ represents a hydrogen atom, a lower alkyl group, an aralkyl group, or a hydroxy group or an ester thereof; and/or
(a3) $R^2$ represents a hydrogen atom or a lower alkyl group; and/or
(a4) $R^3$ represents a halogen atom, a hydrogen atom, a lower alkyl group, or a hydroxy group or an ester thereof; and/or
(a5) $R^4$ represents a halogen atom, a hydrogen atom, a hydroxy group, a lower alkoxy group, an amino group, a lower alkylamino group, or a lower cycloalkylamino group; or
(a6) $R^3$ and $R^4$ may be joined to each other through a nitrogen atom to form an unsaturated [1,2,4]oxadiazine ring; and/or
(a7) $R^5$ represents a hydrogen atom, a lower alkyl group, or a lower cycloalkyl group; and/or
(a8) m represents 0 or 1; and/or
(a9) n represents 0 or 1.

That is, examples of the present compound include compounds and salts thereof which comprise a combination of conditions selected from the above (a1), (a2), (a3), (a4), (a5), [or (a6)], (a7), (a8), and (a9) in the compounds represented by the general formula (1) and salts thereof.
(b) Preferred examples of the present compound include compounds and salts thereof in which the respective groups are groups described below in the compounds represented by the general formula (1) and salts thereof:

(b1) A represents a group represented by the following general formula (p1); and/or

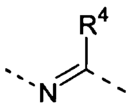
(p1)

(b2) $R^1$ represents a hydrogen atom; and/or
(b3) $R^2$ represents a hydrogen atom; and/or
(b4) $R^3$ represents a hydroxy group or an ester thereof; and/or
(b5) $R^4$ represents a halogen atom; or
(b6) $R^3$ and $R^4$ may be joined to each other through a nitrogen atom to form an unsaturated [1,2,4]oxadiazine ring; and/or
(b7) m represents 0; and/or
(b8) n represents 0 or 1.

That is, preferred examples of the present compound include compounds and salts thereof which comprise one condition or a combination of two or more conditions selected from the above (b1), (b2), (b3), (b4), (b5), [or (b6)], (b7), and (b8) in the compounds represented by the general formula (1).
Incidentally, the compounds and salts thereof which satisfy a combination of the conditions of above (b) and the conditions of the above (a) are also included in the scope of the present compound.

(c) More preferred examples of the present compound include compounds and salts thereof in which the respective groups are groups described below in the compounds represented by the general formula (1) and salts thereof:

(c1) A represents a group represented by the following general formula (p1); and/or

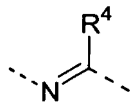

(p1)

(c2) $R^1$ represents a hydrogen atom; and/or
(c3) $R^2$ represents a hydrogen atom; and/or
(c4) $R^3$ represents a hydroxy group; and/or
(c5) $R^4$ represents a chlorine atom; or
(c6) $R^3$ and $R^4$ may be joined to each other through a nitrogen atom to form a 5,6-dihydro-4H-[1,2,4]oxadiazine ring; and/or
(c7) m represents 0; and/or
(c8) n represents 0 or 1.

That is, more preferred examples of the present compound include compounds and salts thereof which comprise one condition or a combination of two or more conditions selected from the above (c1), (c2), (c3), (c4), (c5), [or (c6)], (c7), and (c8) in the compounds represented by the general formula (1).
Incidentally, the compounds and salts thereof which satisfy a combination of the conditions of above (c) and the conditions of the above (a) and/or (b) are also included in the scope of the present compound.
(d) Preferred specific examples of the present compound include the following compounds and salts thereof:

N-(2-hydroxy-3-(1-piperidinyl)propoxy)-pyridine-1-oxide-3-carboximidoyl chloride (hereinafter referred to as "Compound A"),
N-((2R)-2-hydroxy-3-(1-piperidinyl)propoxy)-pyridine-1-oxide-3-carboximidoyl chloride (hereinafter referred to as "Compound B"),
N-(2-hydroxy-3-(1-piperidinyl)propoxy)pyridine-3-carboximidoyl chloride (hereinafter referred to as "Compound C"), and
(-)-5-(piperidin-1-ylmethyl)-3-(pyridin-3-yl)-5,6-dihydro-2H-1,2,4-oxadiazine (hereinafter referred to as "Compound D").

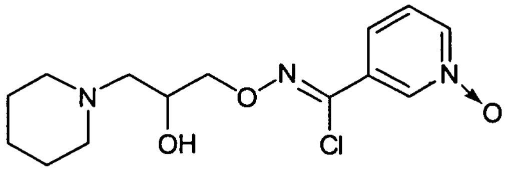

Compound A

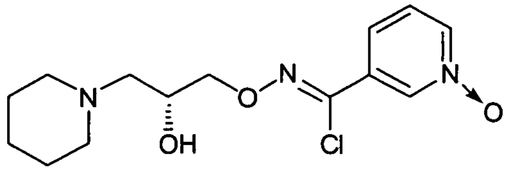

Compound B

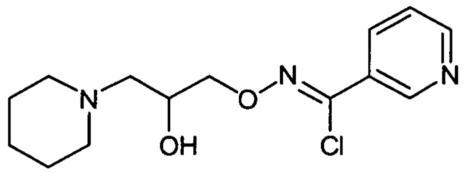

Compound C

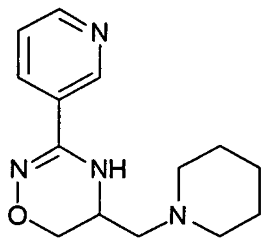

Compound D

Further, a compound in the case where A in the general formula (1) of the present compound is a group represented by the formula (p1):

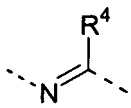

(p1)

is represented by the following general formula.

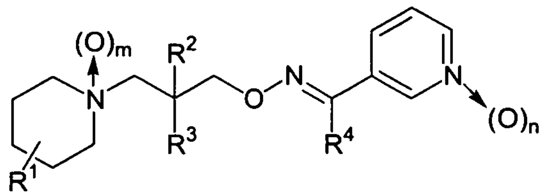

Further, a compound in the case where A in the general formula (1) of the present compound is a group represented by the formula (p2):

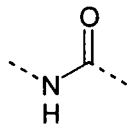

(p2)

is represented by the following general formula.

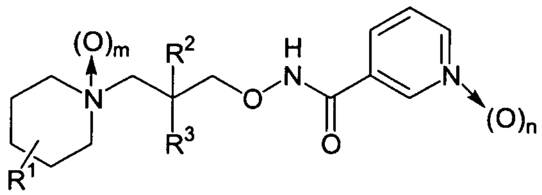

Further, a compound in the case where A in the general formula (1) of the present compound is a group represented by the formula (p3):

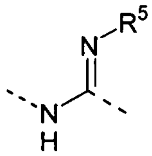

(p3)

is represented by the following general formula.

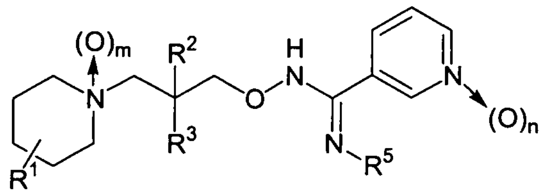

The present compound can be produced according to a common procedure in the field of organic synthetic chemistry. For example, the compound can be produced based on the method described in WO 00/50403, WO 90/04584, WO 00/35914, or US Patent No. 4187220.

The "salt" of the present compound is not particularly limited as long as it is a pharmaceutically acceptable salt, and examples thereof include salts with an inorganic acid such as hydrochloric acid, hydrobromic acid, hydroiodic acid, nitric acid, sulfuric acid, or phosphoric acid; and salts with an organic acid such as acetic acid, fumaric acid, maleic acid,

succinic acid, citric acid, tartaric acid, adipic acid, gluconic acid, glucoheptonic acid, glucuronic acid, terephthalic acid, methanesulfonic acid, lactic acid, hippuric acid, 1,2-ethanedisulfonic acid, isethionic acid, lactobionic acid, oleic acid, pamoic acid, polygalacturonic acid, stearic acid, tannic acid, trifluoromethanesulfonic acid, benzenesulfonic acid, p-toluenesulfonic acid, lauryl sulfate ester, methyl sulfate, naphthalenesulfonic acid, or sulfosalicylic acid; and particularly, a salt with a maleic acid is preferred.

In the invention, examples of the chorioretinal degenerative disease include age-related macular degeneration (drusen formation in early age-related macular degeneration, atrophic age-related macular degeneration, and exudative age-related macular degeneration), retinitis pigmentosa, retinal artery occlusion, retinal vein occlusion, uveitis, Leber's disease, retinopathy of prematurity, retinal detachment, detachment of retinal pigment epithelium, central serous chorioretinopathy, central exudative chorioretinopathy, polypoidal choroidal vasculopathy, multifocal choroiditis, neovascular maculopathy, retinal arterial aneurysm, and an optic nerve disorder accompanying any of these diseases. Preferred examples thereof include age-related macular degeneration and retinitis pigmentosa. In particular, the present compound is useful as a prophylactic or therapeutic agent for drusen formation in early age-related macular degeneration or atrophic age-related macular degeneration.

Further, the present compound can be formulated into a single preparation or a combination preparation by adding a pharmaceutically acceptable additive as needed using a widely used technique.

When the present compound is used for prophylaxis or therapy of the above-mentioned eye disease, it can be administered to a patient orally or parenterally. Examples of the route of administration include oral administration, topical administration to eyes (such as instillation administration, administration into conjunctival sac, intravitreal administration, subconjunctival administration, and sub-Tenon's administration), intravenous administration, and transdermal administration. Further, the present compound is formulated into a dosage form suitable for administration along with a pharmaceutically acceptable additive as needed. Examples of the dosage form suitable for oral administration include tablets, capsules, granules, fine granules, and powders, and examples of the dosage form suitable for parenteral administration include injections, eye drops, ophthalmic ointments, patches, gels, and inserts. These can be prepared using a common technique widely used in this field. Further, the present compound can also be formulated into a preparation for intraocular implant or a DDS (drug delivery system) preparation such as a microsphere other than these preparations.

For example, a tablet can be prepared by properly selecting and using an excipient such as lactose, glucose, D-mannitol, anhydrous calcium hydrogen phosphate, starch, or sucrose; a disintegrant such as carboxymethyl cellulose, carboxymethyl cellulose croscarmellose sodium, crospovidone, starch, partially pregelatinized starch, or low-substituted hydroxypropyl cellulose; a binder such as hydroxypropyl cellulose, ethyl cellulose, gum arabic, starch, partially pregelatinized starch, polyvinyl pyrrolidone, or polyvinyl alcohol; a lubricant such as magnesium stearate, calcium stearate, talc, hydrous silicon dioxide, or a hydrogenated oil; a coating agent such as purified sucrose, hydroxypropylmethyl cellulose, hydroxypropyl cellulose, methyl cellulose, or pyrrolidone; a corrigent such as citric acid, aspartame, ascorbic acid, or menthol; or the like.

An injection can be prepared by selecting and using a tonicity agent such as sodium chloride; a buffer such as sodium phosphate; a surfactant such as polyoxyethylene sorbitan monooleate; a thickening agent such as methyl cellulose; or the like as needed.

An eye drop can be prepared by selecting and using a tonicity agent such as sodium chloride or concentrated glycerin; a buffer such as sodium phosphate or sodium acetate; a surfactant such as polyoxyethylene sorbitan monooleate, polyoxyl 40 stearate, or polyoxyethylene hydrogenated castor oil; a stabilizer such as sodium citrate or sodium edetate; a preservative such as benzalkonium chloride or paraben; or the like as needed. The pH of the eye drop is permitted as long as it falls within the range that is acceptable as an ophthalmic preparation, but is generally preferably in the range of from 4 to 8. Further, an ophthalmic ointment can be prepared with a widely used base such as white petrolatum or liquid paraffin.

An insert can be prepared by pulverizing and mixing a biodegradable polymer such as hydroxypropyl cellulose, hydroxypropylmethyl cellulose, a carboxy vinyl polymer, or polyacrylic acid along with the present compound and compression molding the resulting powder. If necessary, an excipient, a binder, a stabilizer, or a pH adjusting agent can be used.

A preparation for intraocular implant can be prepared using a biodegradable polymer such as polylactic acid, polyglycolic acid, a lactic acid-glycolic acid copolymer, or hydroxypropyl cellulose.

The dose of the present compound can be properly changed depending on the dosage form, severity of symptoms, age or body weight of a patient to which the present compound is to be administered, medical opinion, and the like. In the case of oral administration, the present compound can be generally administered to an adult once or divided into several times at a dose of from 0.01 to 5000 mg, preferably from 0.1 to 2500 mg, more preferably from 0.5 to 1000 mg per day. In the case of an injection, the present compound can be generally administered to an adult once or divided into several times at a dose of from 0.0001 to 2000 mg per day. In the case of an eye drop or an insert, generally a preparation containing the active ingredient in an amount of from 0.000001 to 10% (w/v), preferably from 0.00001 to 1% (w/v), more preferably from 0.0001 to 0.1% (w/v) can be administered once or several times per day. Further, in the

case of a patch, a patch containing the active ingredient in an amount of from 0.0001 to 2000 mg can be applied to an adult, and in the case of a preparation for intraocular implant, a preparation for intraocular implant containing the active ingredient in an amount of from 0.0001 to 2000 mg can be implanted in the eye of an adult.

Advantageous effects of the Invention

As will be described in detail in the section of pharmacological test below, the present compound inhibited photoreceptor cell death and/or visual cell death in a mouse model of light damage. That is, the present compound is useful as a prophylactic or therapeutic agent for a chorioretinal degenerative disease such as age-related macular degeneration (drusen formation in early age-related macular degeneration, atrophic age-related macular degeneration, or exudative age-related macular degeneration), retinitis pigmentosa, retinal artery occlusion, retinal vein occlusion, uveitis, Leber's disease, retinopathy of prematurity, retinal detachment, detachment of retinal pigment epithelium, central serous chorioretinopathy, central exudative chorioretinopathy, polypoidal choroidal vasculopathy, multifocal choroiditis, neovascular maculopathy, retinal arterial aneurysm, or an optic nerve disorder accompanying any of these diseases, preferably for age-related macular degeneration or retinitis pigmentosa, in particular for drusen formation in early age-related macular degeneration or atrophic age-related macular degeneration.

Best Mode for Carrying Out the Invention

Hereinafter, a pharmacological test and preparation examples will be shown, however, these examples are for understanding the invention well, and are not meant to limit the scope of the invention.

[Pharmacological Test]

Test using Mouse Model of Light Damage

By using a mouse model of light damage, the pharmacological effect of a maleate of Compound A (hereinafter referred to as "Compound A' ") was evaluated. Incidentally, the mouse model of light damage comprises model animals in which cell death of retinal photoreceptor cells and/or visual cells was induced by light irradiation, and they are widely used mainly as model animals of a chorioretinal degenerative disease such as age-related macular degeneration or retinitis pigmentosa (Invest. Ophthalmol. Vis. Sci., 2005; 46: 979-987).

(Production Method for Mouse Model of Light Damage and Evaluation Method)

Male BALB/c mice of 8 weeks of age were adapted to the dark for 20 hours in a dark room, and then, light irradiation was performed at 5000 Lux for 2 hours using a white fluorescent lamp, whereby light damage was induced. Thereafter, dark adaptation was performed for 20 hours in a dark room, and the electroretinogram (ERG) was measured. From the obtained waveforms, a- and b-wave amplitudes (μV) were calculated. Incidentally, in a normal control group, ERG was measured after dark adaptation was performed for 20 hours in a dark room without performing light irradiation. The inhibition ratio of amplitude reduction (%) was calculated for both a- and b-wave amplitudes according to the [Equation 3]. Incidentally, the case number in each group is 4 (8 eyes).

[Equation 3]

$$\text{Inhibition Ratio of Amplitude Reduction (\%)} = (V_x - V_0) / (V_n - V_0) \times 100$$

$V_0$: Amplitude in vehicle administration group (μV)
$V_n$: Amplitude in normal control group (μV)
$V_x$: Amplitude in drug administration group (μV)

(Administration Method)

1) In the case of single administration before light irradiation

Compound A' administration group:

A Compound A' solution in which Compound A' was suspended in an aqueous solution of 1% (w/v) methyl cellulose was orally administered once at a dose of 10 mg/kg immediately before light irradiation.

Normal control group and vehicle administration group:

An aqueous solution of 1% (w/v) methyl cellulose was orally administered once at one hour before light irradiation.

(Results)

The test results when Compound A' was used are shown in the following Table 1. As apparent from Table 1, Compound A' exhibited a significant inhibitory effect on the reduction of a- and b-wave amplitudes of ERG by light irradiation.

[Table 1]

| Group | Inhibition ratio of amplitude reduction (%) | |
|---|---|---|
| | a-wave | b-wave |
| Compound A' (10 mg/kg) | 44.5 | 44.5 |

(Discussion)

From the above results, the present compound typified by Compound A' inhibits photoreceptor cell death and/or visual cell death. Therefore, it was shown that the present compound has a prophylactic or improving effect on a chorioretinal degenerative disease.

[Preparation Examples]

The pharmaceutical agent of the invention will be more specifically described with reference to preparation examples, however, the invention is not limited only to these preparation examples.

Formulation Example 1: Tablet

| in 100 mg | |
|---|---|
| Compound A' | 1 mg |
| Lactose | 66.4 mg |
| Cornstarch | 20 mg |
| Carxboxymethyl cellulose calcium | 6 mg |
| Hydroxypropyl cellulose | 6 mg |
| Magnesium stearate | 0.6 mg |

Compound A' and lactose are mixed in a mixer, carboxymethyl cellulose calcium and hydroxypropyl cellulose are added thereto, and the resulting mixture is granulated. The obtained granules are dried, followed by sizing. Then, magnesium stearate is added and mixed with the sized granules and the resulting mixture is tableted with a tableting machine. By changing the addition amount of Compound C, a tablet containing the active ingredient in an amount of 0.1 mg, 10 mg, or 50 mg in 100 mg of tablet can be prepared.

Formulation Example 2: Ophthalmic Ointment

| in 100 g | |
|---|---|
| Compound A' | 0.3 g |
| Liquid paraffin | 10.0 g |
| White petrolatum | q.s. |

Compound A' is added to uniformly melted white petrolatum and liquid paraffin, these ingredients are mixed well, and the resulting mixture is gradually cooled, whereby an ophthalmic ointment is prepared. By changing the addition amount of Compound B, an ophthalmic ointment containing the active ingredient at a concentration of 0.05% (w/w), 0.1% (w/w), 0.5% (w/w) or 1% (w/w) can be prepared.

Formulation Example 3: Injection

| in 10 ml | |
|---|---|
| Compound A' | 10 mg |
| Sodium chloride | 90 mg |
| Polysorbate 80 | q.s. |
| Sterile purified water | q.s. |

Compound A' and sodium chloride are added to sterile purified water, whereby an injection is prepared. By changing the addition amount of Compound A, an injection containing the active ingredient in an amount of 0.1 mg, 10 mg, or 50 mg in 10 ml of injection can be prepared.

Formulation Example 4: Eye drop

| in 100 ml | |
|---|---|
| Compound A' | 10 mg |
| Sodium chloride | 900 mg |
| Polysorbate 80 | q.s. |
| Disodium hydrogen phosphate | q.s. |
| Sodium dihydrogen phosphate | q.s. |
| Sterile purified water | q.s. |

Compound A' and the other above-mentioned ingredients are added to sterile purified water, and these ingredients are mixed well, whereby an eye drop is prepared. By changing the addition amount of Compound A, an eye drop containing the active ingredient at a concentration of 0.05% (w/v), 0.1% (w/v), 0.5% (w/v), or 1% (w/v) can be prepared.

Industrial Applicability

The compound A' which is the active ingredient of the invention, inhibited photoreceptor cell death and/or visual cell death. Therefore, the active ingredient of the invention is useful as a prophylactic or therapeutic agent for a chorioretinal degenerative disease, particularly as a prophylactic or therapeutic agent for age-related macular degeneration, retinitis pigmentosa, or the like.

## Claims

1. A prophylactic or therapeutic agent for a chorioretinal degenerative disease, comprising at least one of a compound

represented by the following general formula (1) or a salt thereof as an active ingredient:

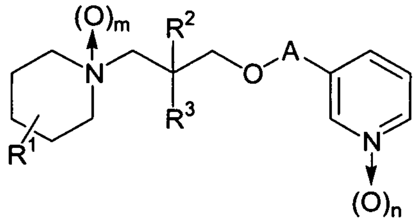

(1)

wherein A represents a group represented by the following general formula (p1), (p2) or (p3);

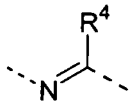

(p1)

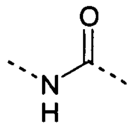

(p2)

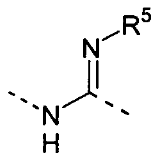

(p3)

$R^1$ represents a hydrogen atom, a lower alkyl group, an aralkyl group, or a hydroxy group or an ester thereof;
$R^2$ represents a hydrogen atom or a lower alkyl group;
$R^3$ represents a halogen atom, a hydrogen atom, a lower alkyl group, or a hydroxy group or an ester thereof;
$R^4$ represents a halogen atom, a hydrogen atom, a hydroxy group, a lower alkoxy group, an amino group, a lower alkylamino group, or a lower cycloalkylamino group; or
$R^3$ and $R^4$ may be joined to each other through a nitrogen atom to form an unsaturated [1,2,4]oxadiazine ring;
$R^5$ represents a hydrogen atom, a lower alkyl group, or a lower cycloalkyl group;
m represents 0 or 1; and
n represents 0 or 1.

**2.** The prophylactic or therapeutic agent according to claim 1, wherein in the general formula (1), A represents a group represented by the following general formula (p1);

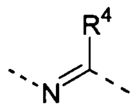

(p1)

$R^1$ represents a hydrogen atom;
$R^2$ represents a hydrogen atom;
$R^3$ represents a hydroxy group or an ester thereof;
$R^4$ represents a halogen atom; or
$R^3$ and $R^4$ may be joined to each other through a nitrogen atom to form an unsaturated [1,2,4]oxadiazine ring;
m represents 0; and
n represents 0 or 1.

**3.** The prophylactic or therapeutic agent according to claim 1, wherein in the general formula (1), A represents a group represented by the following general formula (p1);

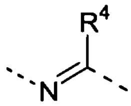

(p1)

$R^1$ represents a hydrogen atom;
$R^2$ represents a hydrogen atom;
$R^3$ represents a hydroxy group;
$R^4$ represents a chlorine atom; or
$R^3$ and $R^4$ may be joined to each other through a nitrogen atom to form a 5,6-dihydro-4H-[1,2,4]oxadiazine ring;
m represents 0; and
n represents 0 or 1.

4. The prophylactic or therapeutic agent according to claim 1, wherein the compound represented by the general formula (1) is:

N-(2-hydroxy-3-(1-piperidinyl)propoxy)-pyridine-1-oxide-3-carboximidoyl chloride,
N-((2R)-2-hydroxy-3-(1-piperidinyl)propoxy)-pyridine-1-oxide-3-carboximidoyl chloride,
N-(2-hydroxy-3-(1-piperidinyl)propoxy)-pyridine-3-carboximidoyl chloride, or
(-)-5-(piperidin-1-ylmethyl)-3-(pyridin-3-yl)-5,6-dihydro-2H-1,2,4-oxadiazine.

5. The prophylactic or therapeutic agent according to any one of claims 1 to 4, wherein the chorioretinal degenerative disease is age-related macular degeneration, retinitis pigmentosa, retinal artery occlusion, retinal vein occlusion, uveitis, Leber's disease, retinopathy of prematurity, retinal detachment, detachment of retinal pigment epithelium, central serous chorioretinopathy, central exudative chorioretinopathy, polypoidal choroidal vasculopathy, multifocal choroiditis, neovascular maculopathy, retinal arterial aneurysm, or an optic nerve disorder accompanying any of these diseases.

6. The prophylactic or therapeutic agent according to any one of claims 1 to 4, wherein the chorioretinal degenerative disease is age-related macular degeneration or retinitis pigmentosa.

7. A prophylactic or therapeutic method for a chorioretinal degenerative disease, comprising administering a pharmacologically effective amount of at least one of a compound represented by the following general formula (1) or a salt thereof to a patient:

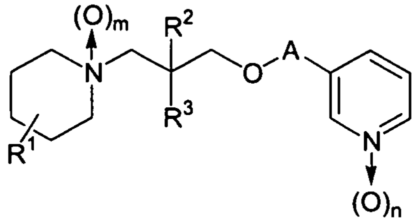

(1)

wherein A represents a group represented by the following general formula (p1), (p2) or (p3);

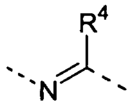

(p1)

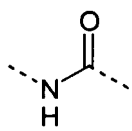

(p2)

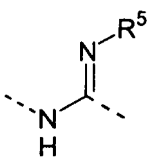

(p3)

$R^1$ represents a hydrogen atom, a lower alkyl group, an aralkyl group, or a hydroxy group or an ester thereof;
$R^2$ represents a hydrogen atom or a lower alkyl group;
$R^3$ represents a halogen atom, a hydrogen atom, a lower alkyl group, or a hydroxy group or an ester thereof;
$R^4$ represents a halogen atom, a hydrogen atom, a hydroxy group, a lower alkoxy group, an amino group, a lower alkylamino group, or a lower cycloalkylamino group; or
$R^3$ and $R^4$ may be joined to each other through a nitrogen atom to form an unsaturated [1,2,4]oxadiazine ring;
$R^5$ represents a hydrogen atom, a lower alkyl group, or a lower cycloalkyl group;
m represents 0 or 1; and

n represents 0 or 1.

**8.** The prophylactic or therapeutic method according to claim 7, wherein in the general formula (1), A represents a group represented by the following general formula (p1);

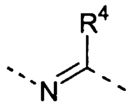

(p1)

$R^1$ represents a hydrogen atom;
$R^2$ represents a hydrogen atom;
$R^3$ represents a hydroxy group or an ester thereof;
$R^4$ represents a halogen atom; or
$R^3$ and $R^4$ may be joined to each other through a nitrogen atom to form an unsaturated [1,2,4]oxadiazine ring;
m represents 0; and
n represents 0 or 1.

**9.** The prophylactic or therapeutic method according to claim 7, wherein in the general formula (1), A represents a group represented by the following general formula (p1);

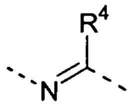

(p1)

$R^1$ represents a hydrogen atom;
$R^2$ represents a hydrogen atom;
$R^3$ represents a hydroxy group;
$R^4$ represents a chlorine atom; or
$R^3$ and $R^4$ may be joined to each other through a nitrogen atom to form a 5,6-dihydro-4H-[1,2,4]oxadiazine ring;
m represents 0; and
n represents 0 or 1.

**10.** The prophylactic or therapeutic method according to claim 7, wherein the compound represented by the general formula (1) is:

N-(2-hydroxy-3-(1-piperidinyl)propoxy)-pyridine-1-oxide-3-carboximidoyl chloride,
N-((2R)-2-hydroxy-3-(1-piperidinyl)propoxy)-pyridine-1-oxide-3-carboximidoyl chloride,
N-(2-hydroxy-3-(1-piperidinyl)propoxy)-pyridine-3-carboximidoyl chloride, or
(-)-5-(piperidin-1-ylmethyl)-3-(pyridin-3-yl)-5,6-dihydro-2H-1,2,4-oxadiazine.

**11.** The prophylactic or therapeutic method according to any one of claims 7 to 10, wherein the chorioretinal degenerative disease is age-related macular degeneration, retinitis pigmentosa, retinal artery occlusion, retinal vein occlusion, uveitis, Leber's disease, retinopathy of prematurity, retinal detachment, detachment of retinal pigment epithelium, central serous chorioretinopathy, central exudative chorioretinopathy, polypoidal choroidal vasculopathy, multifocal choroiditis, neovascular maculopathy, retinal arterial aneurysm, or an optic nerve disorder accompanying any of these diseases.

**12.** The prophylactic or therapeutic method according to any one of claims 7 to 10, wherein the chorioretinal degenerative disease is age-related macular degeneration or retinitis pigmentosa.

**INTERNATIONAL SEARCH REPORT**

International application No.
PCT/JP2009/069526

A. CLASSIFICATION OF SUBJECT MATTER
*A61K31/4545*(2006.01)i, *A61K31/5395*(2006.01)i, *A61P27/02*(2006.01)i

According to International Patent Classification (IPC) or to both national classification and IPC

B. FIELDS SEARCHED

Minimum documentation searched (classification system followed by classification symbols)
A61K31/4545, A61K31/5395, A61P27/02

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2010 |
| Kokai Jitsuyo Shinan Koho | 1971-2010 | Toroku Jitsuyo Shinan Koho | 1994-2010 |

Electronic data base consulted during the international search (name of data base and, where practicable, search terms used)
CA/REGISTRY/MEDLINE/BIOSIS(STN)

C. DOCUMENTS CONSIDERED TO BE RELEVANT

| Category* | Citation of document, with indication, where appropriate, of the relevant passages | Relevant to claim No. |
|---|---|---|
| A | WO 2008/137149 A1 (CYTRX CORP.), 13 November 2008 (13.11.2008), claims; description, paragraphs [0051] to [0057], [0165] to [0194] & US 2009/227572 A1 | 1-6 |
| A | WO 2008/070010 A2 (CYTRX CORP.), 12 June 2008 (12.06.2008), claims; description, paragraphs [0063] to [0069], [0178] to [0216] & TW 200838522 A & IN 200902136 P2 & AU 2007328280 A1 & EP 2089033 A2 & US 2009/233917 A1 | 1-6 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 05 January, 2010 (05.01.10) | 19 January, 2010 (19.01.10) |

| Name and mailing address of the ISA/ | Authorized officer |
|---|---|
| Japanese Patent Office | |
| Facsimile No. | Telephone No. |

Form PCT/ISA/210 (second sheet) (April 2007)

**INTERNATIONAL SEARCH REPORT**

International application No. PCT/JP2009/069526

C (Continuation). DOCUMENTS CONSIDERED TO BE RELEVANT

| Category* | Citation of document, with indication, where appropriate, of the relevant passages | Relevant to claim No. |
|---|---|---|
| A | WO 2000/050403 A1 (BIOREX KUTATO ES FEJLESZTO RT.), 31 August 2000 (31.08.2000), claims; description, page 1, lines 21 to 25; page 4, lines 5 to 21; page 11, line 6 to page 13, line 19; page 16, line 27 to page 17, the last line; table 8 & AU 200031824 A & NO 200104103 A & BR 200008969 A & SK 200101158 A3 & EP 1163224 A1 & CZ 200103053 A3 & KR 2001102410 A & ZA 200106488 A & JP 2002-537384 A & EP 1163224 B1 & DE 60002187 E & US 6649628 B1 & ES 2193055 T3 & AU 779096 B2 & RU 2250901 C2 & IN 200100785 P2 & NO 319793 B1 & CZ 297386 B6 & IL 144866 A & KR 676124 B1 & SK 287063 B6 | 1-6 |

Form PCT/ISA/210 (continuation of second sheet) (April 2007)

**INTERNATIONAL SEARCH REPORT**

International application No.
PCT/JP2009/069526

**Box No. II Observations where certain claims were found unsearchable (Continuation of item 2 of first sheet)**

This international search report has not been established in respect of certain claims under Article 17(2)(a) for the following reasons:

1. [X] Claims Nos.: 7-12
because they relate to subject matter not required to be searched by this Authority, namely:
Claims 7 to 12 include the methods for treatment of the human body or animal body by surgery or therapy.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.
2. [ ] As all searchable claims could be searched without effort justifying additional fees, this Authority did not invite payment of additional fees.
3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest**

[ ] The additional search fees were accompanied by the applicant's protest and, where applicable, the payment of a protest fee.

[ ] The additional search fees were accompanied by the applicant's protest but the applicable protest fee was not paid within the time limit specified in the invitation.

[ ] No protest accompanied the payment of additional search fees.

Form PCT/ISA/210 (continuation of first sheet (2)) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0050403 A
- WO 9004584 A
- WO 0035914 A
- US 4187220 A
- WO 2005041965 A


### Non-patent literature cited in the description


- *Invest. Ophthalmol. Vis. Sci.,* 2005, vol. 46, 979-987